# EUROPEAN PATENT APPLICATION

(11) **EP 1 435 218 A1**
(43) Date of publication of application: **07.07.2004**
(21) Application number: 02447243.3
(22) Date of filing: 05.12.2002
(51) Int. Cl.: A61B 5/107

(54) **Method and device for locating visceral constrictions**

(71) Applicant: INTERUNIVERSITAIR MICROELEKTRONICA CENTRUM VZW, 3001 Leuven (BE); Vantrappen, Gaston, 3090 Overijse (BE)
(72) Inventor: Vantrappen, Gaston, 3090 Overijse (BE)
(74) Representative: Van Malderen, Joelle

(57) **Abstract**

The present invention is related to a method and a device for determining the location of a visceral constriction in a human or animal body, said device comprising :
- a catheter (1), to be inserted into said human or animal body, said catheter being equipped with an element (2, 15,30) the volume of which is variable and dependent on the compression of said element,
- a means for detecting the volume changes of said element,
- a means (10,11) for detecting the position of said element with respect to a predefined reference position, measured along the length of said catheter.

## Description

### Field of the invention

The present invention is related to the determination of the location of an anatomical or functional constriction of a hollow body organ, such as the lower esophageal sphincter (LES) in the gastroesophageal junctional segment, or constrictions in other tubular organs of the body such as other parts of the gastrointestinal tract, the pancreatico-biliary tract, the genito-urinary tract, and blood vessels.

### Background of the invention

The lower esophageal sphincter (LES) has a crucial role in the prevention of gastroesophageal reflux (GER) and of the pathological effects thereof. The term gastroesophageal reflux refers to the backflow of gastric contents, particularly of gastric acid and bile, from the stomach, into the esophagus. When this reflux occurs too often or lasts too long, it results in symptoms and lesions of gastroesophageal reflux disease (GERD).

The quantitative evaluation of gastroesophageal reflux is based on measurement of intraesophageal acidity and bile concentration, using devices (such as pH electrodes, acid exposure sensors, and bile sensors) that have to be positioned at a defined distance above the upper border of the LES. Therefore, the first step in the quantification of abnormal gastroesophageal reflux is the determination of the location of the upper border of the lower esophageal sphincter.

Determining the location of a constriction in a hollow organ is a problem which is applicable to other organs as well. In general, it is often necessary for the physician to identify and indicate the location of a visceral constriction as the number of centimetres an exploring device has to be introduced into the body in order to reach the site of the constriction in the visceral tube, because that is the spatial reference for the application of the diagnostic investigation or therapeutic procedure which is to be performed once the location has been determined.

The term 'constriction' means a localised decrease of the lumen of a hollow organ or a localised decrease of distensibility of such an organ. Such a constriction may be anatomical or functional. An anatomical constriction is caused by a fibrotic, edematous, inflammatory, tumoral or plaque-forming process in the wall of the hollow organ or it may be due to normal structures or pathological processes in the tissues surrounding the said organ. A functional constriction is due to the contractile state of the muscles of the hollow organ, such as that which occurs in sphincteric segments.

In the specific case of the gastro-esophageal functional segment, several techniques have been proposed to locate the lower esophageal sphincter (LES). The radiological identification of the LES requires a fluoroscopic examination by a specialised radiologist and imposes ionising radiation upon the patient. Moreover, the visualisation and location of the LES on radiological images does not allow to position an exploring device at a defined level above the sphincter, unless the device is introduced and positioned in the esophagus during the radiological examination.

Endoscopy frequently does not allow to determine the upper limit of the lower esophageal sphincter. Moreover, endoscopy is an invasive and specialised examination.

The pH step-up technique, such as it is described in the publication by Mattox et al., Digestive Diseases and Sciences, Vol. 37, No. 8 (August 1992), pp. 1185-1191, has been shown to locate the proximal margin of the LES inaccurately.

The wall motion device was developed to detect those gastric antrum contractions that substantially narrow the lumen of the antrum without resulting in an occlusion of that lumen. The device is described in 'Evaluation of patterns of human antral and pyloric motility with an antral wall motor detector', American Journal of Physiology, 1990;258;G616-G623. The most important drawbacks of the wall motion device for determining constrictions in a hollow organ are : firstly, that it measures the diameter changes in a given plane of hollow organ, not the volume changes, and, therefore, will fail to detect a flattening of the lumen which leaves the diameter of that lumen unchanged in the plane of the detector; secondly that its dimensions are such that they make the introduction through the nose very difficult and often impossible, and thirdly that the technique is too complicated to be used for clinical purposes.

It is believed that the most accurate, presently available way of locating the upper border of the lower esophageal sphincter is by manometric identification of the lower esophageal high pressure zone, which corresponds to the lower esophageal sphincter. Manometry, therefore, is at present the first step in the process of measuring gastroesophageal reflux. Manometry is a technically complex laboratory investigation which is only available in highly specialised centres and is expensive. Even if a pH electrode and a pressure measuring device (solid state or perfused catheter) are combined in a single probe, such as described by S Singh et al., American Journal of Gastroenterology, 1992 ; vol. 87, pp. 967-970, by Devault and Castell, Am J Gastroenterology, 1991, vol. 86, pp. 380-381, by Klingler et al., Am J Gastroenterology, 2000, vol. 95, No. 4, and in document US-A-5117827, the cost and complexity of this examination remains prohibitive. This is the reason why the use of manometric techniques (including the LES locating device described by Singh et all.) is limited to highly specialised centers. Esophageal pH measurement, therefore, is at present not available for a large number of patients with gastroesophageal reflux disease for whom this examination would be very useful.

In general, treatment of constrictions in hollow viscera, due to processes such as fibrotic stricture, tumor growth or plaque formation often require local invasive procedures such as dilation, stenting or irradiation.

So far, accurate location of constrictions in other parts of the gastrointestinal tract, in the biliopancreatic or genitourinary tract or in blood vessels is usually based on techniques similar to the above described techniques for determining the location of the lower esophageal sphincter, namely endoscopy, manometry and radiological or MRI imaging.

Therefore, there is a need for a device that allows to determine visceral constrictions in a simple, non-expensive way, without manometry, so that it becomes accessible to all patients who need it.

### Aim of the invention

The aim of the present invention is to obtain a method and device which may determine the location of a visceral constriction in a hollow organ in a simple and inexpensive way, so that it may be used in a medical office outside a hospital environment, without the help of radiological, endoscopic or manometric techniques.

### Summary of the invention

The present invention is related to a device for determining the location of a visceral constriction in a human or animal body, said device comprising :
- a catheter, to be inserted into said human or animal body, said catheter being equipped with an element the volume of which is variable and dependent on the compression of said element,
- a means for detecting the volume changes of said element,
- a means for detecting the position of said element with respect to a predefined reference position, measured along the length of said catheter.

According to a first embodiment, said compressible element is a balloon filled with a liquid or a gas.

According to this first embodiment, a device of the invention may comprise :
- a catheter,
- a balloon, the interior of which is in connection with the interior of said catheter,
- a container, having a variable content,
- a measurement and read-out device, equipped with a means for detecting said volume change by measuring a physical value related to said volume change, and for displaying and/or recording said physical value,
- a first tube, connecting said catheter to said container,
- a second tube, connecting said catheter to said measurement and read-out device,
- a stopcock, which can be placed in two positions, a first position of which opens up the connection between said balloon and said container, whilst blocking the connection between said balloon and said measurement and read-out device, and a second position of which opens up the connection between said balloon and said measurement and read-out device, whilst blocking the connection between said balloon and said container.

Said container may be a vessel equipped with a piston or said container may be a balloon.

According to a second embodiment, in a device of the invention, said element of variable volume comprises a compressible material, which is electrically conductive, and whereof the electrical resistance is dependent on the compression of said material.

According to this second embodiment, a device of the invention may comprise :
- a catheter,
- said element,
- two electrodes, attached to said element,
- two electrical conductors, each conductor connected to one of said electrodes,
- a measurement and read-out device, connected to said conductors, and equipped with a means for measuring the electrical resistance between said electrodes, as well as displaying and/or recording the value of said resistance.

Still according to this second embodiment, said element may consist of a piece of electrically conductive foam, enveloped by a membrane, or it may consist of a capsule containing electrically conductive granules.

According to a third embodiment, said element of variable volume consists of a closed balloon consisting of a first and second chamber, connected to each other by a tube, a liquid being present in said balloon, one of said chambers comprising a transducer, which is able to detect said liquid being pressed from the other chamber into said one chamber.

According to this third embodiment, the device of the invention may comprise :
- a catheter,
- said balloon, attached to said catheter along the length of said catheter, so that when the catheter is inserted into the body, said first chamber is inserted firstly, and said second chamber is inserted secondly and wherein said balloon is filled with an electrically conductive liquid, and wherein said transducer is present in said second chamber and wherein said transducer is a piece of foam, equipped with two electrodes,
- two electrical conductors, connected to said electrodes,
- a measurement and read-out device, connected to said conductors, and equipped with a means for measuring the electrical resistance between said electrodes, as well as displaying and/or recording the value of said resistance.

According to a fourth embodiment, said element of variable volume comprises a balloon at one end, and a rigid hollow body at the other, said balloon and said rigid body being connected by a tube, and a gas being present inside the space formed by said element, said rigid hollow body further comprising a compressible body, which is separated from said gas by a membrane in such a way that said compressible body can be compressed by gas which is pressed out of said balloon and into said rigid hollow body, and wherein said compressible body comprises a compressible material, which is electrically conductive, and whereof the electrical resistance is dependent on the compression of said material.

According to this fourth embodiment, said material may be an electrically conductive foam, or said material may consist of electrically conductive granules.

Still according to this fourth embodiment, the device of the invention may comprise :
- a catheter,
- said element, attached to said catheter along the length of said catheter, so that when the catheter is inserted into the body, said balloon is inserted firstly, and said rigid hollow body is inserted secondly and wherein said compressible body is equipped with two electrodes,
- two electrical conductors, connected to said electrodes,
- a measurement and read-out device, connected to said conductors, and equipped with a means for measuring the electrical resistance between said electrodes, as well as displaying and/or recording the value of said resistance.

The present invention is equally related to a method for determining the location of a visceral constriction, said method comprising the steps of :
- inserting a device comprising a compressible element according to the invention, into the body, until said compressible element has passed said constriction,
- defining a reference point for the distance over which the device is inserted into the body,
- pulling out the device, while monitoring the volume changes of said compressible element, as a function of the distance the device has been pulled out, said distance being measured with respect to said reference point.

### Brief description of the figures

Figures 1a and 1b are schematic representations of a balloon-catheter based device according to the present invention.

Figure 2 is a schematic representation of a device according to the invention, based on a detector made of compressible foam.

Figure 3 is a schematic representation of a device according to the invention, based on a detector made of a closed liquid-filled balloon.

Figure 4 and 5 are schematic representations of a device according to the invention, based on a gas-filled detector.

Figure 6 shows results of an experiment, comparing a device of the invention to a prior art manometric device.

### Detailed description of the invention

The present invention is related to a device which comprises an element which has a variable volume, a means to pick up the volume changes the element undergoes when it is pulled through a constriction in the body organ, and a measuring/displaying/recording system, to show the volume changes which occur at each of the measuring levels during the pull-through procedure. A device of the invention further comprises a means for measuring the distance over which the element has been pulled.

According to the invention, the volume change detector may be a fluid- or gas filled balloon, a spongelike compressible body or another structure which is able to undergo volume changes when it is pulled through a constriction in a hollow organ of the body and to re-assume its original volume when it is out of that constriction. The measuring system may be contained within the volume detector from where it is connected to an external (out of the body) displaying system. The measuring system may also be separate from the volume change detector but connected to it, inside or outside the body. The measuring system is also connected to the displaying system which shows the volume changes measured at various levels of the constriction during the pull-through procedure, thus allowing to localize the constriction in the hollow organ as the distance (e.g. in centimetres) from the port of entry into the body onto the margins of the constriction.

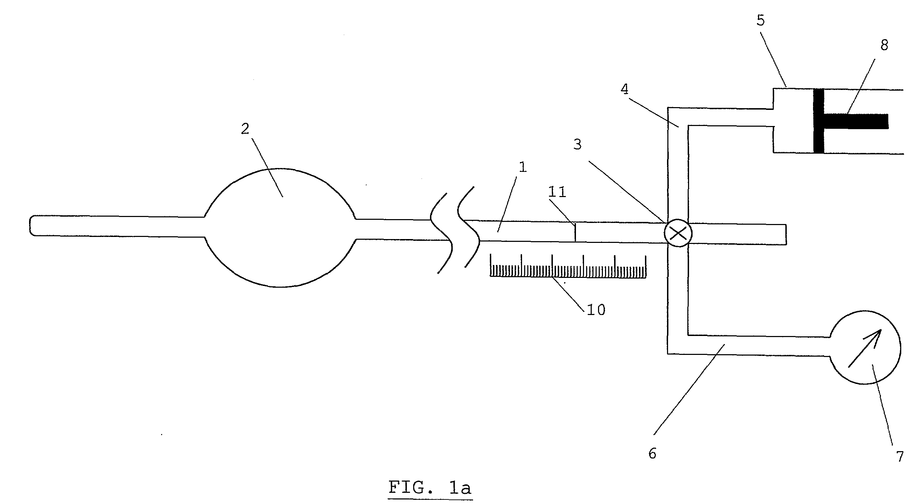

Figure 1a shows a device according to a first embodiment of the invention. The device comprises a catheter 1, connected to a balloon 2, the interior of which is in connection with the interior of the catheter, i.e. the balloon and catheter enclose a continuous hollow space. The catheter is equipped with a three-way stopcock 3, which is connected by a first connecting tube 4 to a container 5 with variable content, and by a second connecting tube 6 to a measurement and read-out device 7. A liquid is present in the device as shown in figure 1a. The container 5 may be a vessel equipped with a piston 8, which is the case in the figure 1a. The measurement and read-out device 7 may for example comprise a vertical liquid column, connected to the catheter and balloon, so that a volume change of the balloon may be translated into a change in liquid column height.

The stopcock 3 can be put in a first position, connecting the interior of the catheter 1 and balloon 2 with the interior of the container 5, while blocking the connection of the catheter 1 to the measurement and read-out device 7. In that way, the liquid may be induced to flow from the container to the balloon and vice versa. By moving the stopcock 3 to a second position, the interior of the catheter 1 and balloon 2 can be put in connection with the measurement and read-out device 7, while blocking the connection between the balloon 2 and the container 5.

The device operates in a straightforward way. Before insertion of the device, the liquid contained in the balloon 2 is induced to flow into the container 5, by the piston movement or by compression of balloon 2, thereby emptying the balloon 2. Deflating the balloon in this way facilitates insertion of the catheter and balloon into the body. For LES-location, the catheter would be inserted through the nose of a patient, sufficiently far for the balloon to enter the stomach. At that point, the balloon is filled with liquid from the container 5 through the piston action, with the stopcock 3 in the first position. Then the stopcock 3 is moved to the second position, connecting the filled balloon with the measurement and read-out device 7. After that, the catheter and balloon are pulled out slowly. The device is equipped with a means for measuring the distance the balloon has been pulled out. This may be a simple ruler 10, along which the catheter is moved during the pull-through action, in combination with a marking 11 on the catheter, so that the distance over which the catheter has been pulled out can be recorded with respect to a reference position, for example the position wherein the marking 11 coincides with the beginning of the ruler 10. Of course, other and more sophisticated means for measuring the pull-out distance may be implemented as well, but it is imperative that this distance can be monitored. During the pull-through action, the balloon enters the LES at some point. The diameter of the liquid-filled balloon is larger than the diameter of the LES, so that the balloon is compressed when it is pulled through the closed LES, leading to a change in its volume. Liquid is pressed out of the balloon as a consequence, and flows towards the read-out device 7. Compression of the balloon is thereby detected, for example through a rise in height of a liquid column, at which point it is established that the balloon has entered the LES. When the pull-through procedure is continued, the balloon undergoes further compression, as it passes the LES. Leaving the LES, the balloon reassumes its former volume, which is detected as a decrease in liquid column height. The device of the invention allows thus to detect the position of the LES, by recording the volume changes indicated on the read-out device, as a function of the pull-through distance. More specifically, the device allows to locate the upper border of the LES as a sharp decrease in the height of a liquid column, caused by the sudden return of the volume detector, i.e. the balloon, to the volume it had before entering the constriction formed by the LES.

The device may be used to detect any other visceral constriction. The dimensions of the device (length and diameter of catheter, dimensions of the balloon, etc..) will have to be adapted to every specific case.

The balloon is made of a thin membrane. This balloon has a length of a few millimetres to a few centimetres, depending upon the nature and location of the constriction. The maximum diameter of the balloon is usually from three to ten millimetres.

Instead of a piston-equipped vessel, the container 5 can be a second balloon. It is imperative that the container's volume can be increased or decreased as required by the procedure which has to be performed. In other words, it has to be able to accommodate the liquid pressed out of the balloon which is being pulled through a constriction. The volume of the external container 5 is such that it easily allows to receive the maximum volume of liquid contained in the detecting balloon 2.

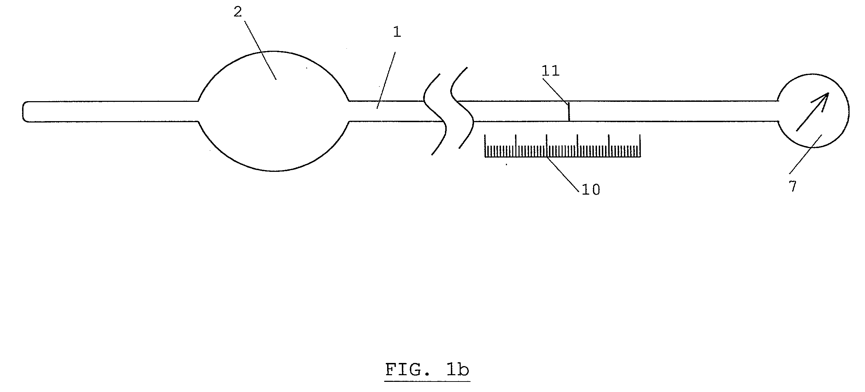

In a simplified embodiment, the container 5 may be omitted from the device. This requires that the balloon can be inserted up to and through the constriction portion while the liquid is inside the balloon. In the case of the measurement device being based on a liquid column, this is normally possible. In this simplified embodiment (figure 1b), the stopcock is equally left out, and there is a direct connection between the balloon 2 and the measurement and read-out device 7.

The catheter 1 is made of a material which has a low compliance and is sufficiently flexible to be easily introduced in the hollow organ but, at the same time, sufficiently stiff in order to be pushed up or down the hollow organ. The catheter may be stiffened by an appropriate means, for example a guiding wire (not shown in the figures), so that it can be easily pushed up into the hollow organ of the body that is to be examined. The length of the catheter is adapted to the distance of the constriction in the hollow organ from the port of entry into the body and can vary from a few centimetres to one meter or more. The external diameter of the catheter is adapted to the nature of the organ which is to be explored but usually varies between two and four millimetres.

The measurement and read-out device 7 may be working according to the principle of a liquid column, such as described above. However, any other device capable of measuring, directly or indirectly, the volume changes the detecting balloon undergoes when it is pulled through the constriction, may be implemented, in combination with a means for displaying and/or recording the measured value during the pull-through procedure. Generally, in a device of the invention, these volume changes may be transduced into changes in electrical, optical or other physical variables which are a measure of said volume changes and which can be transmitted to an external read-out or recording device.

The device shown in figure 1 is filled with an amount of liquid which is sufficient to allow the measuring device 7 to detect and measure the volume changes which occur in the internal balloon (e.g. when it is pulled through a constriction in the hollow organ which is being examined). This amount of liquid depends upon the size of the internal balloon 2 and upon the length and diameter of the catheter 1.

In stead of filling the detecting balloon of figures 1a and 1b with a liquid, this balloon can be filled with gas and connected via a catheter to an external measurement and read-out device. In the latter case, the measuring and read-out device 7 outside the body may consist of a device which is capable of measuring the volume of gas pushed out of the detecting balloon when this balloon passes a constriction. As an example, the measuring device may consist of an electrically conductive foam, which, when compressed by gas coming out of the detecting balloon, undergoes changes in electrical resistance that are determined by the degree of compression.

According to another embodiment, the detector does not consist of a fluid-filled balloon but of another type of compressible body, the volume changes of which are transduced into changes in electrical, optical or other physical variables which can be transmitted to an external read-out device.

In one preferred embodiment, the compressible body is made of a piece of an electrically conductive foam or sponge or a capsule containing electrically conductive granules. Any compressible body used in this embodiment of the invention is characterized by high compressibility and low compression set, the latter meaning that the compressible body will return to its original volume after compression. Suitable elastomeric materials are e.g. conductive polyurethane or silicone. The material used must have an electrical resistance which is dependent on the degree of compression. Conductive foam products exist, for example foams whose electrical resistance decreases with compression.

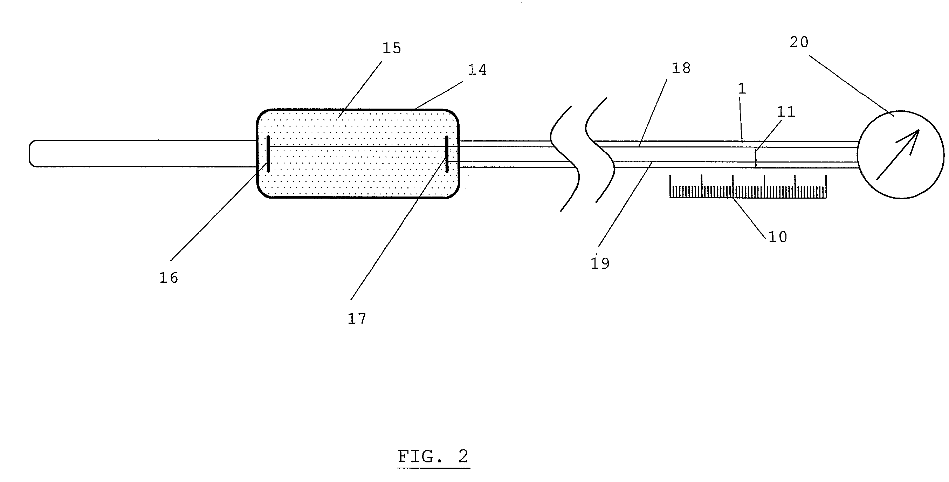

Figure 2 shows a schematic view of a device according to this embodiment. At the end of a catheter 1, a cylinder-shaped piece of conductive foam 15, the detector, is attached to the catheter or incorporated into said catheter. The piece of foam 15 is preferably encapsulated by a membrane 14, to avoid that the foam would absorb fluids. Two electrodes 16 and 17 are attached to the detector 15, at the extremities of this cylinder-shaped detector. Conducting wires 18 and 19 are present inside the catheter, connecting the electrodes 16 and 17 to an external measurement and read-out device 20. The latter device comprises at least a means for measuring the resistance between the electrodes 16 and 17. Any known device for measuring such a resistance value may be implemented, as well as a suitable device for displaying the measured value. As in the previous embodiment, a ruler 10 and marking 11 or any equivalent device is present, to detect the distance over which the catheter has been pulled out.

The procedure is also similar : insertion of the device, for example through the nose, until the detector 15 has passed the visceral constriction, for example the LES. Then a slow pull-through procedure, wherein the electrical resistance value is recorded, for example every centimeter. Barring any significant compression of the detector, the resistance value remains virtually constant. When the detector 15 enters the constriction portion, a change in electrical resistance will be recorded. After the constriction portion, the resistance will return to its original value. The resistance change allows to locate the position of the visceral constriction.

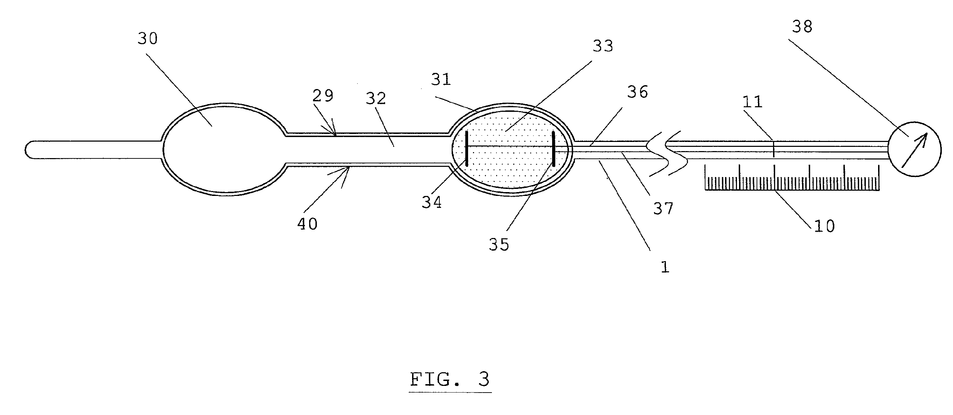

According to another embodiment, shown in figure 3, the device of the invention comprises a detector in the form of a closed balloon 29 consisting of two chambers 30 and 31, connected by a channel 32. The balloon is at least partially filled with a liquid and inserted in or otherwise connected to a catheter 1. When the device enters the body of a patient, the first chamber 30, which we call the distal chamber, enters first, followed by the second, 'proximal' chamber 31. The proximal chamber comprises a transducer which is able to detect the liquid that is being pressed from the distal chamber into the proximal chamber, when the distal chamber passes a constriction. In one preferred embodiment, the balloon is filled with an electrically conductive liquid, while the proximal chamber comprises a transducer in the form of a piece of foam 33, which practically fills that chamber 31. The foam is preferably of high compressibility and low compression set, and is equipped with two electrodes 34 and 35, connected by wires 36 and 37 to an external measurement and read-out device 38. The piece of foam 33 in this embodiment is possibly but not necessarily electrically conductive. When the liquid is pushed into the proximal chamber, the foam 33 is impregnated by the liquid, which influences the electrical resistance of the piece of foam 33. This change is picked up by the measurement device and displayed and/or recorded. The balloon is attached to a catheter 1, for example by incorporating the balloon into a distal portion 40 of said catheter, said portion having the same or similar shape as the balloon.

The localisation procedure takes place in the same way as described above. The catheter is inserted until both the distal and the proximal chambers of the balloon 29 have passed the constriction to be detected. In this way, the distal chamber 30 is emptied when it is pushed through the constriction, and subsequently filled when the proximal chamber 31 passes through the constriction. Then a pull-through procedure commences, in conjunction with a means 10,11 for measuring the pull-out distance. The proximal chamber 31 is once more compressed during the pull-through, making sure thereby that the distal chamber 30 is sufficiently filled with liquid before said distal chamber is pulled through the constriction. This pulling through of the distal chamber 30 then allows a change in resistance to be recorded, which can then be translated into the location of the visceral constriction.

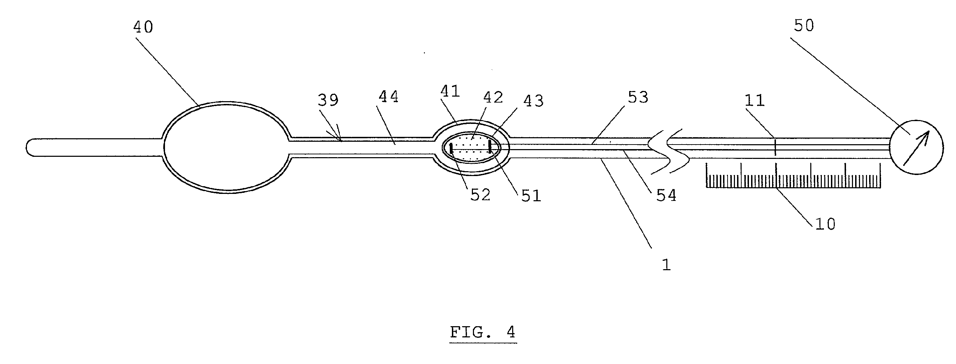

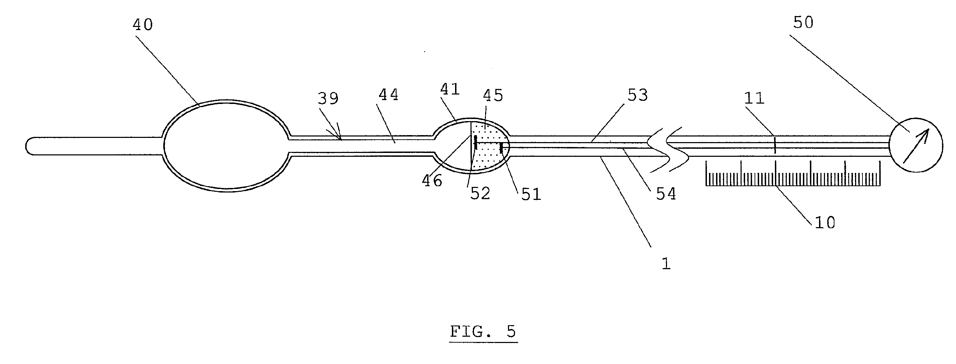

In stead of using a liquid-filled detector 29, a gas-filled detector 39 may be used (see figure 4). In this embodiment, the distal chamber is a compressible balloon 40, while the proximal chamber is a rigid capsule 41, containing a body which can be compressed by gas, such as a piece of electrically conductive foam 42 enveloped by a thin membrane 43. It is preferred that the proximal chamber 41 is sufficiently small in diameter so that it can be pulled through the constriction. The distal and proximal chamber are connected by a tube 44 to form one hollow body 39. In a slightly alternative form shown in figure 5, a piece of foam 45 may be used which is filling the proximal part of the capsule 41 but which is separated from the gas coming from the compressed distal chamber 40 by a thin membrane 46. Preferably, the compressible body (42 or 45) in the proximal chamber should be of high compressibility and low compression set. The compressible body (42 or 45) has an electrical resistance that is dependent on the degree of compression, which in turn depends on the volume of gas compressing it. The piece of foam is connected to the measurement and read-out system 50 through electrodes (51, 52) and conductors (53, 54), in an analogue way as in previous embodiments (figures 2 and 3). The localisation procedure is the same as the one described for the device of figure 3.

In the embodiments of figures 2 to 5, the catheter has the same characteristics than in the balloon-equipped device, and may be produced from the same materials.

A device according to the invention may differ from the embodiments described and shown in the drawings without leaving the scope of the invention. For example, the distance between electrodes 16,17 or 34,35 or 51, 52 may vary. More than two electrodes might be present, and/or the electrodes might be placed circumferentially around the foam element 15 in stead of placed at either end of the element. Also, this element is not limited to a cilinder-like shape.

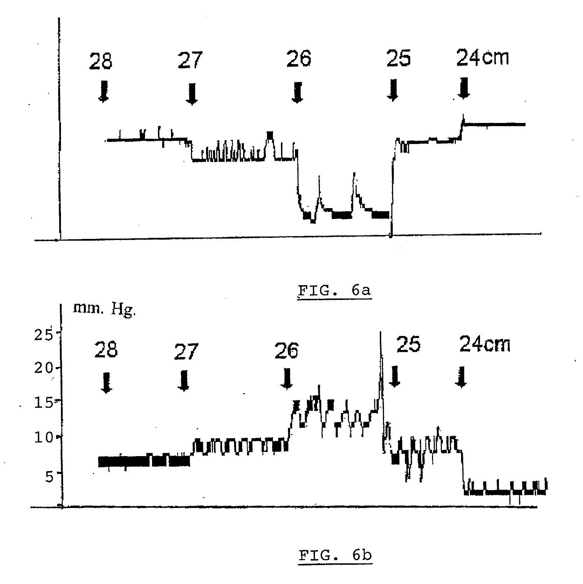

As an illustration of the performance of a device of the invention, reference is made to figure 6a and 6b. The graphs show the results of an experiment performed on an anesthetised cat. The lower graph (fig. 6b) shows a manometric recording of the lower esophegeal high pressure zone. The pressures were measured using a stepwise pull-through technique and a Dent sleeve (Ref. Dent J. Am J Gastroenterology, 1976, vol 71, pp. 263-267). The distances indicated in cm describe the distance d between the cat's incisors and the manometric device. The LES is located between 27 and 24 cm from the incisors.

The upper graph (fig. 6a) shows a volumetric recording performed with a balloon-equipped device of the invention such as the one shown in figure 1a. The measurement was performed with an endoluminal electrical impedance catheter with ring electrodes spaced at intervals of 1cm (Ref. 'Verification of the intraluminal multiple electrical impedance measurement for the recording of gastro-intestinal motility', Silny et al, Journal of Gastro-intestinal motility, 1993, 5, p 107-122. This device was used in order to translate the height of the fluid column into the read-out signal which is shown in the graph. The recording represents, in a qualitative way, the volume changes the balloon undergoes when it is pulled from the stomach, at 28cm from the cat's incisors, through the lower esophageal sphincter into the esophagus, at 24cm from the incisors. The zone of decreased volume extends from 27 cm to 24cm from the incisors and corresponds well with the 'high pressure zone', manometrically determined in the same cat during the same experiment (fig. 6b).

It can be concluded from the experiment illustrated in figure 6 that the LES is located with the same accuracy, using the device of the invention, than it is using a manometric device, in particular a Dent sleeve.

## Claims

1. A device for determining the location of a visceral constriction in a human or animal body, said device comprising :
- a catheter (1), to be inserted into said human or animal body, said catheter being equipped with an element (2, 15,29,39) the volume of which is variable and dependent on the compression of said element,
- a means for detecting the volume changes of said element,
- a means (10,11) for detecting the position of said element with respect to a predefined reference position, measured along the length of said catheter.

2. The device according to claim 1, wherein said compressible element is a balloon (2) filled with a liquid or a gas.

3. The device according to claim 2, wherein said device comprises :
- a catheter (1),
- a balloon (2), the interior of which is in connection with the interior of said catheter (1),
- a container (5), having a variable content,
- a measurement and read-out device (7), equipped with a means for detecting said volume change by measuring a physical value related to said volume change, and for displaying and/or recording said physical value,
- a first tube (4), connecting said catheter (1) to said container (5),
- a second tube (6), connecting said catheter (1) to said measurement and read-out device (7),
- a stopcock (3), which can be placed in two positions, a first position of which opens up the connection between said balloon (2) and said container (5), whilst blocking the connection between said balloon (2) and said measurement and read-out device (7), and a second position of which opens up the connection between said balloon (2) and said measurement and read-out device (7), whilst blocking the connection between said balloon (2) and said container (5).

4. The device according to claim 1, 2 or 3 wherein said container (5) is a vessel equipped with a piston (8).

5. The device according to claim 1, 2 or 3 wherein said container (5) is a balloon.

6. The device according to claim 1, wherein said element (15) comprises a compressible material, which is electrically conductive, and whereof the electrical resistance is dependent on the compression of said material.

7. The device according to claim 6, wherein said device comprises :
- a catheter (1),
- said element (15),
- two electrodes (16,17), attached to said element,
- two electrical conductors (18,19), each conductor connected to one of said electrodes (16,17),
- a measurement and read-out device (20), connected to said conductors (18,19), and equipped with a means for measuring the electrical resistance between said electrodes (16,17), as well as displaying and/or recording the value of said resistance.

8. The device according to claim 6 or 7, wherein said element (15) consists of a piece of electrically conductive foam, enveloped by a membrane (14).

9. The device according to claim 6 or 7, wherein said element (15) consists of a capsule containing electrically conductive granules.

10. The device according to claim 1, wherein said element (29) consists of a closed balloon consisting of a first and second chamber (30,31), connected to each other by a tube (32), a liquid being present in said balloon, one of said chambers comprising a transducer (33), which is able to detect said liquid being pressed from the other chamber into said one chamber.

11. The device according to claim 10, wherein said device comprises :
- a catheter (1),
- said balloon (29), attached to said catheter along the length of said catheter, so that when the catheter is inserted into the body, said first chamber (30) is inserted firstly, and said second chamber (31) is inserted secondly and wherein said balloon (29) is filled with an electrically conductive liquid, and wherein said transducer is present in said second chamber and wherein said transducer is a piece of foam (33), equipped with two electrodes (34,35),
- two electrical conductors (36,37), connected to said electrodes (34,35),
- a measurement and read-out device (38), connected to said conductors (36,37), and equipped with a means for measuring the electrical resistance between said electrodes (34,35), as well as displaying and/or recording the value of said resistance.

12. The device according to claim 1, wherein said element (39) comprises a balloon (40) at one end, and a rigid hollow body (41) at the other, said balloon and said rigid body being connected by a tube (44), and a gas being present inside the space formed by said element (39), said rigid hollow body (41) further comprising a compressible body (42,45), which is separated from said gas by a membrane (43,46) in such a way that said compressible body can be compressed by gas which is pressed out of said balloon (40) and into said rigid hollow body (41), and wherein said compressible body (42,45) comprises a compressible material, which is electrically conductive, and whereof the electrical resistance is dependent on the compression of said material.

13. The device according to claim 12, wherein said material is an electrically conductive foam.

14. The device according to claim 12, wherein said material consists of electrically conductive granules.

15. The device according to any one of claims 12 to 14, wherein said device comprises :
- a catheter (1),
- said element (39), attached to said catheter along the length of said catheter, so that when the catheter is inserted into the body, said balloon (40) is inserted firstly, and said rigid hollow body (41) is inserted secondly and wherein said compressible body (42,45) is equipped with two electrodes (51,52),
- two electrical conductors (53,54), connected to said electrodes (51,52),
- a measurement and read-out device (50), connected to said conductors (53,54), and equipped with a means for measuring the electrical resistance between said electrodes (51,52), as well as displaying and/or recording the value of said resistance.

16. A method for determining the location of a visceral constriction, said method comprising the steps of :
- inserting a device comprising a compressible element (2,15,29,39) according to any one of the preceding claims, into the body, until said compressible element has passed said constriction,
- defining a reference point for the distance over which the device is inserted into the body,
- pulling out the device, while monitoring the volume changes of said compressible element, as a function of the distance the device has been pulled out, said distance being measured with respect to said reference point.
